(19) Europäisches Patentamt
European Patent Office
Office européen des brevets

(11) Veröffentlichungsnummer: **0 018 540**
**B1**

# (12) EUROPÄISCHE PATENTSCHRIFT

(45) Veröffentlichungstag der Patentschrift: **14.10.81**

(51) Int. Cl.³: **C 07 C 143/68,** C 07 F 9/12, C 07 C 35/21

(21) Anmeldenummer: **80102013.2**

(22) Anmeldetag: **15.04.80**

(54) **Ester des Isocamphyl-guajakols, Verfahren zu ihrer Herstellung und ihre Verwendung zur Herstellung von 3-(Iso-camphyl-(5))-cyclohexanol.**

(30) Priorität: **28.04.79 DE 2917360**

(43) Veröffentlichungstag der Anmeldung: **12.11.80 Patentblatt 80/23**

(45) Bekanntmachung des Hinweises auf die Patenterteilung: **14.10.81 Patentblatt 81/41**

(84) Benannte Vertragsstaaten: **CH DE FR GB IT LI NL**

(56) Entgegenhaltungen:
**DE-B-1 223 482**
**CHEMICAL ABSTRACTS, Band 66, Nr. 15, 10. April 1967, Seite 6186, Nr. 65644v**
**Columbus, Ohio, U.S.A.**
**L.A. KHEIFITS et al.: »Study of terpenophenols. XXVIII. Products of condensation of camphene with guaiacol and their hydration«**
**CHEMICAL ABSTRACTS, Band 68, Nr. 9, 26. Februar 1968, Seite 3878, Nr. 39823z**
**Columbus, Ohio, U.S.A.**
**I.S. AUL'CHENKO et al.: »Terepene phenols. XXIX. Synthesis of m-terpene phenols«**

(73) Patentinhaber: **HAARMANN & REIMER GMBH, Postfach 138, D-3450 Holzminden (DE)**

(72) Erfinder: **Bauer, Kurt, Dr., Corveyblick 41, D-3450 Holzminden (DE)**
Erfinder: **Lange, Gerd-Karl, Dr., Am Hungerborn 4, D-3450 Holzminden (DE)**

(74) Vertreter: **Dill, Erwin, Dr. et al, c/o BAYER AG Zentralbereich Patente Marken und Lizenzen Bayerwerk, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Ester des Isocamphyl-guajakols, Verfahren zu ihrer Herstellung und ihre Verwendung zur Herstellung von 3-[Isocamphyl-(5)]-cyclohexanol

Die Erfindung betrifft [Isocamphyl-(5)]-guajacyl-ester der Formel

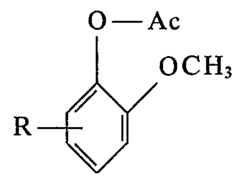

(I)

in der

R für einen in 2- oder 4-Stellung zur OAc-Gruppe befindlichen Isocamphyl-(5)-Rest und
Ac für einen $C_1-C_4$-Alkylsulfonyl- oder Di-($C_1-C_4$-alkyl)-phosphoryl-Rest

stehen.

Die Erfindung betrifft ferner ein Verfahren zur Herstellung der Ester der Formel (I). Das Verfahren ist dadurch gekennzeichnet, daß man 2- oder 4-[Isocamphyl-(5)]-guajakol mit einem Säurechlorid der Formel

$$AcCl \qquad (II)$$

in der

Ac die unter Formel (I) angegebene Bedeutung hat,

in Gegenwart eines Säurebindemittels umsetzt.

Außerdem betrifft die Erfindung die Verwendung der Ester der Formel (I) zur Herstellung von 3-[Isocamphyl-(5)]-cyclohexanol.

Als $C_1-C_4$-Alkylreste seien der n-Propyl-, i-Propyl-, n-Butyl- und vor allem der Methyl- und Ethylrest genannt.

Bevorzugte Ester der Formel (I) sind der O,O-Diethyl-O-6-[isocamphyl-(5)]-guajacyl- und vor allem der O,O-Diethyl-O-4-[isocamphyl-(5)]-guajacyl-phosphorsäureester.

Zur Herstellung der erfindungsgemäßen Ester der Formel (I) werden 6- oder 4-[Isocamphyl-(5)]-guajakol mit $C_1-C_4$-Alkylsulfonsäure- oder Di-($C_1-C_4$-Alkyl)-phosphorsäurechlorid in Gegenwart von Säurebindemitteln umgesetzt. Isocamphyl-(5)-guajakol und Säurechlorid werden vorteilhaft in einem Molverhältnis von 1 : 1—5, vorzugsweise 1 : 1—1,5 angewendet.

Als Säurebindemittel kommen vorzugsweise Stickstoffbasen, insbesondere Triethylamin und Pyridin, in Frage. Die Stickstoffbasen können zugleich als Lösungsmittel dienen, wenn sie in einer über die zum Binden der entstehenden Säure hinausgehenden Menge verwendet werden. Die Acylierung kann aber auch in Gegenwart anderer unter den Reaktionsbedingungen inerter Lösungsmittel wie Benzol, Toluol, Aceton, Diethylether oder Dioxan durchgeführt werden.

Die Acylierung wird vorteilhaft bei 0 bis 50° C, vorzugsweise 5 bis 20° C, vorgenommen.

Als besonders vorteilhaft zur Herstellung der Dialkylphosphorsäureester hat es sich erwiesen, die Phosphorsäurechloride in situ aus Tetrachlorkohlenstoff und den entsprechenden Dialkylphosphiten herzustellen. Dabei wird das Isocamphyl-(5)-guajakol in mindestens der äquivalenten Menge Tetrachlorkohlenstoff gelöst. Dann werden nacheinander Dialkylphosphit und Trialkylamin in einer Menge von 1 bis 5 Mol, vorzugsweise 1 bis 1,1 Mol, je Mol Isocamphyl-(5)-guajakol zugegeben. Überschüssiger Tetrachlorkohlenstoff kann als Lösungsmittel dienen. Als Lösungsmittel können aber auch andere chlorierte Kohlenwasserstoffe, z. B. Methylenchlorid, Chloroform, Trichlorethylen oder Dichlorethylen zugesetzt werden.

Die erfindungsgemäßen Ester fallen bei ihrer Herstellung im allgemeinen bereits in so reiner Form an, daß sie ohne Reinigung unmittelbar weiter verarbeitet werden können.

Zur Weiterverarbeitung zum 3-[Isocamphyl-(5)]-cyclohexanol werden die erfindungsgemäßen Ester der Formel (I) zunächst reduktiv zum 3-[Isocamphyl-(5)]-anisol gespalten.

Die Alkylsulfonsäureester werden durch katalytische Hydrierung in Gegenwart eines Säurebindemittels reduktiv gespalten. Die Hydrierung kann bei Normaldruck oder erhöhtem Druck bis zu 10 Atmosphären vorgenommen werden. Vorzugsweise wird bei Normaldruck gearbeitet. Als Katalysatoren werden feinverteilte Edelmetalle wie Palladium, Rhodium oder Ruthenium, vorzugsweise Palladium, die auf Träger wie Kohle aufgebracht wurden, verwendet. Die Katalysatoren werden bei der Hydrierung in einer Menge von 0,05 bis 2 Gew.-%, vorzugsweise 0,5 bis 1,5 Gew.-%, Edelmetall bezogen auf das Gewicht des Alkylsulfonats eingesetzt.

Zum Abfangen der bei der Hydrierung entstehenden Alkylsulfonsäure wird mindestens 1 Mol Säurebindemittel, vorzugsweise eine Stickstoffbase wie Triethylamin oder Pyridin, zugesetzt. Überschüssige Stickstoffbase kann gleichzeitig auch als Lösungsmittel dienen. Es können aber auch andere unter den Reaktionsbedingungen inerte Lösungsmittel, z. B. Alkohole wie Methanol, verwendet werden. Die Reduktion wird vorzugsweise bei Raumtemperatur durchgeführt.

Die Dialkylphosphate werden durch Behandeln mit Lösungen von Alkalimetallen wie Lithium, Natrium oder Kalium, in flüssigem Ammoniak reduktiv gespalten. Die Alkalimetalle werden in Mengen von mindestens 2 bis 4 Mol, vorzugsweise 2,5 bis 3 Mol, je Mol Dialkylphosphat eingesetzt. Flüssiger Ammoniak wird in 1 bis 10 Gew.-Teilen, vorzugsweise 1,5 bis 4 Gew.-Teilen, je Gew.-Teil Dialkylphosphat verwendet. Es hat sich als vorteilhaft erwiesen, den Phosphorsäureester in Diethylether gelöst zur Lösung des Alkalimetalles in Ammoniak zuzutropfen. Die Spaltung der Dialkylphosphorsäureester wird bei −20 bis −80°C, vorzugsweise −40 bis −50°C, vorgenommen, wobei bei einer Temperatur über −35°C unter Druck gearbeitet werden muß.

Nach beendeter Spaltung der Dialkylphosphate wird ein Elektronenfänger, wie z. B. Natriumbenzoat, zugesetzt, um die Bildung von Dihydroverbindungen zu verhindern. Anschließend wird das gebildete Alkaliamid durch Zugabe von Ammoniumchlorid zersetzt.

Das bei der reduktiven Spaltung der erfindungsgemäßen Ester der Formel (I) anfallende 3-[Isocamphyl-(5)]-anisol wird nach dem in C.A. 68 (1968), 39 823 z beschriebenen Verfahren durch mehrstündiges Erhitzen auf 180 bis 220°C in geschmolzenem Pyridinhydrochlorid in 3-[Isocamphyl-(5)]-phenol überführt und dieses mit Wasserstoff in Gegenwart eines Edelmetallkatalysators zu 3-[Isocamphyl-(5)]-cyclohexanol hydriert.

Die erfindungsgemäßen Ester der Formel (I) sind wertvolle Zwischenprodukte zur Herstellung von 3-[Isocamphyl-(5)]-cyclohexanol, einem wichtigen Inhaltsstoff des Sandelkörpers. Mit ihrer Hilfe läßt sich 3-[Isocamphyl-(5)]-cyclohexanol in wesentlich wirtschaftlicherer Weise als nach den bisher bekannten Verfahren herstellen.

Die in C. A. 68 (1968), 39 823 z als Zwischenprodukte für die Herstellung des 3-[Isocamphyl-(5)]-cyclohexanols vorgeschlagenen 2,4-Dinitrophenylether des 6- und 4-[Isocamphyl-(5)]-guajakols liefern 3-[Isocamphyl-(5)]-phenol, die letzte Vorstufe bei der Herstellung des 3-[Isocamphyl-(5)]-cyclohexanols, nur in etwa 50%iger Ausbeute bezogen auf 4-[Isocamphyl-(5)]-guajakol. Mit Hilfe der erfindungsgemäßen Ester der Formel (I) wird dagegen 3-[Isocamphyl-(5)]-phenol in bis zu 86%iger Ausbeute bezogen auf 4-[Isocamphyl-(5)]-guajakol erhalten. Außerdem sind die erfindungsgemäßen Ester unvergleichlich viel billiger als die 2,4-Dinitrophenylether, zu deren Herstellung das schwierig zugängliche und teure 2,4-Dinitrofluorbenzol benötigt wird.

Das in der DE-AS 1 223 482 vorgeschlagene Verfahren zur Herstellung von 3-[Isocamphyl-(5)]-cyclohexanol ist noch wesentlich unwirtschaftlicher als das in C. A. 68 (1968), 39 823 z beschriebene Verfahren. Es geht von Terpenylphenolen aus, die in einer komplizierten Vielstufen-Reaktion über Terpenyl-methylketone in 3-[Isocamphyl-(5)]-cyclohexen-(2)-on-(1) überführt werden, was dann zum 3-[Isocamphyl-(5)]-cyclohexanol hydriert wird. Die Ausbeute beträgt bei diesem Verfahren noch nicht einmal 20% bezogen auf das Ausgangs-terpenyl-phenol.

## Beispiel 1

130 g (0,5 Mol) 4-[Isocamphyl-(5)]-guajakol werden unter Erwärmen in 43,5 g (0,55 Mol) Pyridin gelöst. Zu dieser Lösung werden innerhalb von 30 Minuten 63 g (0,55 Mol) Methansulfonsäurechlorid unter Rühren und Kühlen so zugetropft, daß die Innentemperatur nicht über 15 bis 20°C steigt. Das Reaktionsgemisch wird 12 Stunden bei 5 bis 10°C aufbewahrt; anschließend wird es mit 100 ml Ether und 100 ml Wasser versetzt. Die organische Phase wird abgetrennt, mit verdünnter Salzsäure gewaschen, anschließend getrocknet und vom Lösungsmittel befreit. Rückstand: 178 g.

Das Rohprodukt wird durch Umfällen aus Ether/Petrolether (Kp. 30 bis 40°C) gereinigt. Bei −20°C kristallisieren 139 g Methansulfonsäure-4[Isocamphyl-(5)]-guajacylester aus.

Durch Einengen der Mutterlauge und Animpfen werden weitere 3 g Ester erhalten; Gesamtausbeute an Ester: 142 g (= 84% der Theorie); Fp.: 65°C.

## Beispiel 2

260 g (1 Mol) 4-[Isocamphyl-(5)]-guajakol werden in 260 ml Tetrachlorkohlenstoff gelöst. Die Lösung wird bei 20°C unter Rühren tropfenweise mit 151,8 g (1,1 Mol) Diethylphosphit versetzt. Anschließend werden unter starkem Kühlen 113 g (1,1 Mol) Triethylamin zugetropft. Nach etwa 5stündigem Rühren bei Raumtemperatur wird die Reaktionsmischung mit verdünnter Salzsäure und anschließend mit verdünnter Natronlauge gewaschen. Die organische Phase wird abgetrennt, getrocknet und vom Lösungsmittel befreit.

Ausbeute: 400 g (= 100% der Theorie) O,O-Diethyl-O-4[Isocamphyl-(5)]-guajacylphosphat; Kp.: 160°C/0,2 Torr.

Verwendungsbeispiel

a[1]) 120 g (0,35 Mol) Methansulfonsäure-4-[Isocamphyl-(5)]-guajacylester (hergestellt nach Beispiel 1) werden in 800 ml Methanol gelöst. Die Lösung wird nach Zugabe von 36 g (0,355 Mol) Triethylamin und 12 g 5%iger Palladiumkohle mit Wasserstoff unter einem Druck von einer Atmosphäre hydriert. Nach etwa 6 Stunden, wenn 75% der theoretisch benötigten Wasserstoffmenge aufgenommen ist, werden weitere 12 g 5%ige Palladiumkohle zugesetzt und so lange weiterhydriert, bis kein Wasserstoff mehr aufgenommen wird.

Nach dem Abfiltrieren des Katalysators wird das Filtrat zur Trockne eingeengt. Der Rückstand wird in Ether gelöst. Die Etherlösung wird zum Entfernen der Salze gewaschen. Nach dem Abdestillieren des Ethers wird der Rückstand destilliert.

Ausbeute: 74 g (= 86,7% der Theorie) 3-[Isocamphyl-(5)]-anisol; Kp.: 160° C/1 Torr.

a[2]) Die Lösung von 1500 g O,O-Diethyl-O-4[Isocamphyl-(5)]-guajacylphosphat (hergestellt nach Beispiel 2) in 1500 ml Diethylether wird bei −40° C langsam unter Rühren zu einer Lösung von 200 g Natrium in 4 l flüssigem Ammoniak getropft. Gegen Ende der Reaktion werden weitere 20 g Natrium zugesetzt. Die tiefblaue Reaktionslösung wird anschließend nacheinander zunächst mit 120 g Natriumbenzoat, dann mit 350 g Ammoniumchlorid versetzt. Dann läßt man den Ammoniak abdampfen, nimmt den Rückstand in Toluol auf und wäscht die Toluollösung mit Wasser. Das Toluol wird im Vakuum abgezogen und der Rückstand destilliert.

Ausbeute: 840 g (= 90,8% der Theorie) 3-[Isocamphyl-(5)]-anisol; Kp.: 131 bis 139° C/0,7 Torr.

b) 2300 g (9,4 Mol) 3-[Isocamphyl-(5)]-anisol werden bei 130° C in 3260 g (58,2 Mol) geschmolzenes Pyridinhydrochlorid unter Rühren eingetragen. Die Mischung wird nach Zugabe von 300 ml Eisessig 3 Stunden auf 190° C erhitzt. Nach dem Abkühlen wird die Reaktionsmischung in Wasser gegeben und das entstandene Gemisch mit Toluol extrahiert. Die Toluollösungen werden bereinigt und vom Toluol befreit. Der verbleibende Rückstand wird destilliert.

Ausbeute: 2072 g (= 95,6% der Theorie) 3-[Isocamphyl-(5)]-phenol; Kp.: 142° C/0,6 Torr.

c) 690 g (3 Mol) 3-[Isocamphyl-(5)]-phenol werden in 360 ml Methanol gelöst und in Gegenwart von 34 g Rutheniumkatalysator (5% Ruthenium auf Aluminiumoxid) unter einem Wasserstoffdruck von 200 Atmosphären bei 140° C hydriert. Nach 3 Stunden ist die Wasserstoffaufnahme beendet. Der Katalysator wird abfiltriert und das Filtrat vom Lösungsmittel befreit. Der Rückstand wird destilliert.

Ausbeute: 686,5 g (= 97% der Theorie) 3-[Isocamphyl-(5)]-cyclohexanol (cis-trans-Isomeren-Gemisch); Kp.: 130 bis 135° C/0,2 Torr.

**Patentansprüche**

1. [Isocamphyl-(5)]-guajacylester der Formel

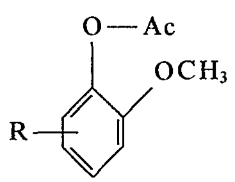

in der

R für einen in 2- oder 4-Stellung zur OAc-Gruppe befindlichen Isocamphyl-(5)-Rest und
Ac für einen $C_1-C_4$-Alkylsulfonyl- oder Di-($C_1-C_4$-alkyl)-phosphoryl-Rest

stehen.

2. O,O-Diethyl-O-4-[isocamphyl-(5)]-guajacyl-phosphat.

3. Verfahren zur Herstellung von [Isocamphyl-(5)]-guajacyl-estern der Formel

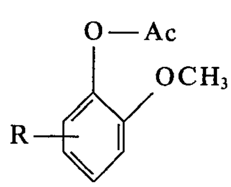

in der

R für einen in 2- oder 4-Stellung zur OAc-Gruppe befindlichen Isocamphyl-(5)-Rest und
Ac für einen $C_1-C_4$-Alkylsulfonyl- oder Di-($C_1-C_4$-alkyl)-phosphoryl-Rest

stehen, dadurch gekennzeichnet, daß man 2- oder 4-[Isocamphyl-(5)]-guajakol mit einem Säurechlorid der Formel

AcCl

in der

Ac die vorstehend angegebene Bedeutung hat,

in Gegenwart eines Säurebindemittels umsetzt.

4. Verwendung der [Isocamphyl-(5)]-guajacyl-ester gemäß Anspruch 1 und 2 zur Herstellung von 3-[Isocamphyl-(5)]-cyclohexanol.

## Claims

1. [Isocamph-5-yl]-guaiacyl esters of the formula

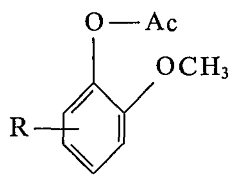

in which

R represents an isocamph-5-yl radical located in the 2-position or 4-position relative to the OAc group and
Ac represents a $C_1-C_4$-alkylsulphonyl or die-($C_1-C_4$-alkyl)-phosphoryl radical.

2. O,O-Diethyl O-4-[isocamph-5-yl]-guaiacyl phosphate.

3. Process for the preparation of [isocamph-5-yl]-guaiacyl esters of the formula

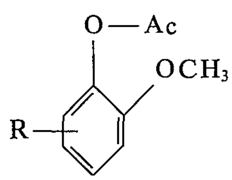

in which

R represents an isocamph-5-yl radical located in the 2-position or 4-position relative to the OAc group and
Ac represents a $C_1-C_4$-alkylsulphonyl or die-($C_1-C_4$-alkyl)-phosphoryl radical,

characterised in that 2- or 4-[isocamph-5-yl]-guaiacol is reacted with an acid chloride of the formula

AcCl

in which

Ac has the meaning indicated above,

in the presence of an acid-binding agent.

4. Use of the [isocamph-5-yl]-guaiacyl esters according to Claim 1 and 2 for the preparation of 3-[isocamph-5-yl]-cyclohexanol.

**Revendications**

1 Esters [isocamphyl-(5)] gaïacyliques de formule

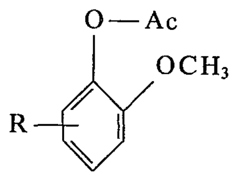

dans laquelle

R représente un reste isocamphyle-(5) en position 2 ou 4 par rapport au groupe OAc, et
Ac représente un groupe alkylsulfonyle en $C_1 - C_4$ ou die-(alkyl en $C_1 - C_4$)-phosphoryle.

2 Le phosphate de O,O diéthyle et de O-4-[isocamphyl-(5)]-gaïacyle.

3 Procedé de préparation des esters [isocamphyl-(5)]-gaïacyliques de formule

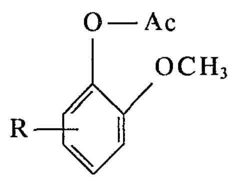

dans laquelle

R représente un reste isocamphyle-(5) en position 2 ou 4 par rapport au groupe OAc et
Ac représente un groupe alkylsulfonyle en $C_1 - C_4$ ou di-(alkyl en $C_1 - C_4$)-phosphoryle,

caracterisé en ce que l'on fait réagir le 2 ou le 4 [isocamphyl-(5)] gaïacol avec un chlorure d'acide de formule

AcCl

dans laquelle

Ac a la signification indiquée ci dessus,

en présence d'un agent fixant les acides

4. Utilisation des esters [isocamphyl-(5)]-gaïacyliques selon les revendications 1 et 2 pour la préparation du 3-[isocamphyl-(5)]-cyclohexanol.